(19) Europäisches Patentamt

European Patent Office

Office européen des brevets

(11) Veröffentlichungsnummer: **0 460 478 B1**

(12) **EUROPÄISCHE PATENTSCHRIFT**

(45) Veröffentlichungstag der Patentschrift: **08.11.95**

(21) Anmeldenummer: **91108515.7**

(22) Anmeldetag: **25.05.91**

Die Akte enthält technische Angaben, die nach
dem Eingang der Anmeldung eingereicht wurden
und die nicht in dieser Patentschrift enthalten sind.

(51) Int. Cl.⁶: **A61K 6/10**

(54) **Lichthärtende Abformmaterialien.**

(30) Priorität: **07.06.90 DE 4018183**

(43) Veröffentlichungstag der Anmeldung:
**11.12.91 Patentblatt 91/50**

(45) Bekanntmachung des Hinweises auf die
Patenterteilung:
**08.11.95 Patentblatt 95/45**

(84) Benannte Vertragsstaaten:
**AT BE CH DE DK ES FR GB GR IT LI LU NL SE**

(56) Entgegenhaltungen:
**EP-A- 0 204 160**
**EP-A- 0 212 681**
**EP-A- 0 255 286**
**EP-A- 0 269 071**
**FR-A- 2 391 705**

(73) Patentinhaber: **BAYER AG**

**D-51368 Leverkusen (DE)**

(72) Erfinder: **Müller, Hanns Peter, Dr.**
**Weizenfeld 36**
**W-5060 Bergisch Gladbach 2 (DE)**
Erfinder: **Voigt, Reiner**
**An der Lichtenburg 4**
**W-5090 Leverkusen (DE)**
Erfinder: **Winkel, Jens, Dr.**
**Letterhaus Strasse 1**
**W-5000 Köln 81 (DE)**
Erfinder: **Schwabe, Peter, Dr.**
**Dudweiler Strasse 17**
**W-5090 Leverkusen 1 (DE)**

**Beschreibung**

Die Erfindung betrifft lichthärtbare Abformmaterialien für dentale Anwendungen. Die im Rahmen der vorliegenden Erfindung beschriebenen Abformmassen dienen zur Herstellung von Abdrücken im Dentalbereich, z. B. bei der Herstellung von Inlays, Kronen, Brücken und Prothesen. Mit ihrer Hilfe kann ein Negativ der Kiefersituation hergestellt werden, welches anschließend, z. B. mit Modellgips, ausgegossen wird. Das so erhaltene Modell wird als Grundlage für die weiteren Arbeiten beim Zahntechniker verwandt. Um eine ausreichende Paßgenauigkeit der vom Techniker hergestellten Werkstücke zu garantieren, müssen die verwendeten Abformmassen eine möglichst geringe Dimensionsänderung zeigen.

Es werden eine Reihe von Materialien für diese Anwendung verwendet: z. B. Polysulfide, Polyether, kondensations- und additionsvernetzende Siloxane. Alle diese Materialien sind Zwei-Komponenten-Systeme, bei denen nach Angaben des Herstellers in einem bestimmten Gewichts- bzw. Volumenverhältnis eine Basismit einer Katalysatorpaste innerhalb von 30 bis 40 Sekunden homogen gemischt und in weiteren, maximal 90 Sekunden, die Mischung auf die abzuformenden Partien plaziert werden muß. Fehlerhafte Handhabungen, wie ungenaues Dosieren und ungenügendes Mischen der Komponenten sowie das Einarbeiten von Luftblasen kann zu unbrauchbaren Abformungen führen. Bei den in der vorliegenden Erfindung beschriebenen Materialien handelt es sich um pastöse Ein-Komponenten-Materialien, welche direkt aus einer lichtundurchlässigen Tube oder Kartusche auf den Abformlöffel gegeben oder auf die abzuformende Partie gespritzt werden können und anschließend unter der Einwirkung von Licht - vorzugsweise mit einer Wellenlänge zwischen 300 und 600 nm - zu einem gummielastischen Abdruck vernetzt werden.

Lichthärtende Abformmaterialien sind bereits bekannt. Verglichen mit den in US 4,575,545 beschriebenen Silikonacrylate zeigen die erfindungsgemäßen Materialien deutlich verringerte Inhibitionsschichten. Im Vergleich zu den in den Europäischen Patentanmeldungen 257 777, 255 286 und 269 071 beschriebenen Urethandiacrylaten zeigen die erfindungsgemäßen höher funktionellen Acrylate überraschenderweise eine geringere Dimensionsänderung. Das ist für die Verwendung als dentales Abformmaterial ein wesentlicher Vorteil.

EP-A-204 160 beschreibt die Verwendung UV-härtbarer Lacke auf Basis linearer polyacrylat-terminierter Polyurethane zur Beschichtung von optischen Glasfasern.

Ein Problem bei der Abformung von Zähnen wird häufig dadurch verursacht, daß dünne Filme des Abformmaterials abreißen und in den Zahnfleischtaschen verbleiben. Diese Rückstände können schwere Entzündungen hervorrufen. Dieses Problem kann aufgrund der hohen Reißfestigkeit der erfindungsgemäßen Abformmaterialien durch deren Verwendung ebenfalls gelöst werden.

Es wurde nun gefunden, daß bei Verwendung von Zubereitungen enthaltend lichthärtenden Prepolymeren aus jeweils wenigstens einem Makrodiol, vorzugsweise einem Polyetherdiol des Molekulargewichtsbereichs 400-20.000, einem aliphatischen Polyisocyanat gegebenenfalls niedermolekularen Diolen bzw. Diaminkettenverlängerern,ungesättigte Endgruppen enthaltende Monoalkohole als Kettenabbrecher und üblichen Hilfs- und Zusatzmitteln, dadurch gekennzeichnet, daß die mittlere Funktionalität der Prepolymeren bezogen auf die ungesättigten Gruppen > 2 ist zur Herstellung von Abformmaterialien in der Dentaltechnik diese die Nachteile der Produkte des Standes der Technik nicht aufweisen.

Bevorzugt werden hierzu solche lichthärtenden Prepolymere verwendet, bei denen als Kettenabbrecher Hydroxyacrylate bzw. -methacrylate, Glycerindiacrylat, Glycerindimethacrylat, Glycerinacrylatmethacrylat, Pentaerythrittriacrylat, Pentaerythttrimethacrylat bzw. gemischte Ester des Pentaerythrits mit Acrylsäure und Methacrylsäure gegebenenfalls in Abmischung mit Hydroxyethylacrylat bzw. - methacrylat eingesetzt werden mit der Maßgabe, daß die mittlere Funktionalität der Prepolymeren > 2 ist.

Insbesondere bevorzugt sind solche, bei denen zum Aufbau der ungesättigten Endgruppen enthaltenden Prepolymeren aliphatische und/oder cycloaliphatische Diisocyanate, gegebenenfalls im Gemisch mit Tri- und Tetraisocyanaten eingesetzt werden mit der Maßgabe, daß die mittlere Funktionalität der Prepolymeren bezogen auf die ungesättigten Gruppen >2 ist.

Es wurde auch ein Verfahren zur Herstellung von lichthärtenden Abformmaterialien gefunden, das dadurch gekennzeichnet ist, daß pro 1 Mol Makrodiol des MG Bereichs >400 gegebenenfalls in Abmischung mit niedermolekularen Diolen des MG Bereichs 62-400, 1,7 bis 3, bevorzugt 1,8 bis 2,2, Mol Diisocyanat(mischung),verwendet werden und das erhaltene Zwischenprodukt mit Hydroxyethylacrylat bzw. -methacrylat und OH-gruppenhaltigen Poly(meth)acrylsäureestern zu Prepolymeren umgesetzt werden, deren mittlere Funktionalität bezogen auf die ungesättigten Gruppen >2 ist.

Die erfindungsgemäß verwendbaren Urethanacrylate enthalten mindestens drei Acrylsäure- bzw. Methacrylsäureester. Sie können hergestellt werden, indem man aliphatische und/oder cycloaliphatische Diisocyanate mit Makrodiolen z.B. Dihydroxypolyethern des mittleren Molgewichtsbereiches $M_n$ von 400 bis 6000 umsetzt, wobei man gegebenenfalls zusätzlich noch aliphatische und/oder cycloaliphatische zweiwertige Alkohole eines mittleren Molgewichtes Mn von 62 bis <400 zusetzt, und die erhaltenen Prepolymeren mit Hydroxyalkylacrylaten bzw. -methacrylaten umsetzt, wobei man gegebenenfalls noch aliphatisch und/oder cycloaliphatische Diamine mit primären Aminogruppen mit einem Molekulargewicht von Mn von 60 bis 300 mitverwenden kann, indem man

a) Hydroxyalkylpolyacrylate oder Hydroxyalkylpolymethacrylate oder gemischte Hydroxyalkylacrylate -methacrylate allein oder in Abmischung mit Hydroxyethylacrylat bzw. methacrylat zusetzt, wobei man ferner

b) pro 1 Mol Makrodiol gegebenenfalls in Abmischung mit makromolekularen Diolen und/oder Diaminen
- 1,7 bis 3, bevorzugt 1,8 bis 2,2 Mol Diisocyanat(mischung)

einsetzt.

Die Umsetzung der Komponenten erfolgt bei Temperaturen von 20 bis 150 °C, bevorzugt von 60 bis 120 °C.

Die gegebenenfalls eingesetzten Diamine dienen zur Einstellung des jeweils gewünschten Molekulargewichtes.

Geeignete Diisocyanate sind insbesondere solche mit aliphatisch und/oder cycloaliphatisch gebundenen Isocyanatgruppen der Formel $Q(NCO)_2$, in welcher Q für einen aliphatischen Kohlenwasserstoffrest mit 2 bis 12 Kohlenstoffatomen oder einen cycloaliphatischen bzw. gemischt aliphatisch-cycloaliphatischen Kohlenwasserstoffrest mit 4 bis 15 Kohlenstoffatomen steht.

Beispiele derartiger Diisocyanate sind Ethylendiisocyanat, Tetramethylendiisocyanat, Hexamethylendiisocyanat, Dodecamethylendiisocyanat, Cyclobutan-1,3-diisocyanat, Cyclohexan-1,3- und 1,4-diisocyanat oder 1-Isocyanato-3,3,5-trimethyl-5-isocyanatomethyl-cyclohexan, 4,4'-Diisocyanato-dicyclohexylmethan bzw. beliebige Gemische derartiger Diisocyanate. Vorzugsweise werden beim erfindungsgemäßen Verfahren cycloaliphatische bzw. gemischt aliphatische-cycloaliphatische Diisocyanate eingesetzt. Besonders bevorzugt ist 1-Isocyanato-3,3,5-trimethyl-5-isocyanato-methyl-cyclohexan (Isophorondiisocyanat).

Geeignete Dihydroxypolyether sind ebenfalls solche der an sich bekannten Art und werden z. B. durch Polymerisation von Epoxiden wie Ethylenoxid, Propylenoxid, Butylenoxid, Tetrahydrofuran, Styroloxid oder Epichlorhydrin mit sich selbst, z.B. in Gegenwart von $BF_3$, oder durch Anlagerung dieser Epoxide, gegebenenfalls im Gemisch oder nacheinander, an Startkomponenten mit reaktionsfähigen Wasserstoffatomen wie Alkohole oder Amine, z.B. Wasser, Ethylenglykol, Propylenglykol-(1,3) oder -(1,2), 4,4'-Dihydroxydiphenylpropan, Anilin, hergestellt. Bevorzugt sind solche Polyether, die überwiegend (bis zu 90 Gew.-%, bezogen auf alle vorhandenen OH-Gruppen im Polyether) primäre OH-Gruppen aufweisen.

Geeignete Diamine sind vorzugsweise primäre Aminogruppen aufweisende aliphatische, cycloaliphatische oder gemischt aliphatisch-cycloaliphatische Diamine des Molekulargewichtsbereichs 60 bis 300. Beispiele sind Ethylendiamin, Tetramethylendiamin, Hexamethylendiamin, 4,4'-Diamino-dicyclohexylmethan, 1,4-Diaminocyclohexan, 4,4'-Diamino-3,3'-dimethyl-dicyclohexylmethan oder 1-Amino-3,3,5-trimethyl-5-aminomethyl-cyclohexan (Isophorondiamin). Ganz besonders bevorzugt werden 4,4'-Diaminodicyclohexylmethan oder das zuletzt genannte Isophorondiamin eingesetzt.

Als zweiwertige Alkohole kommen z. B. Ethylenglykol, Propylenglykol-(1,2) und -(1,3), Butylenglykol-(1,4) und -(2,3), Hexandiol-(1,6), Octandiol-(1,8), Neopentylglykol, Cyclohexandimethanol, 1,4-Bis-hydroxymethyl-cyclohexan, 2-Methyl-1,3-propandiol, 3-Methylpentandiol-(1,5), ferner Diethylenglykole, Dipropylenglykol, Polypropylenglykole, Dibutylenglykole und Polybutylenglykole in Frage.

Geeignete Hydroxyacrylate bzw. -methacrylate sind Glycerindiacrylat, Glycerindimethacrylat, Glycerinacrylatmeth-acrylat, Pentaerythrittriacrylat, Pentaerythrittrimethacrylat bzw. gemischte Ester des Pentaerythrits mit Acrylsäure und Methacrylsäure. Erfindungsgemäß eignen sich auch Mischungen der beschriebenen OH-gruppenhaltigen Poly(meth)acrylsäureester mit Hydroxyethylacrylat bzw. -methacrylat.

Erfindungsgemäß eignen sich auch Abmischungen der beschriebenen Urethanacrylate mit prinzipiell allen radikalisch härtbaren Monomeren.

Insbesondere eignen sich hierfür die an sich bekannten Methacrylate in monofunktioneller oder polyfunktioneller Form, die allein oder in Mischungen eingesetzt werden können. Beispiele sind Methylmethacrylat, Isobutylmethacrylat, Cyclohexylmethacrylat, Ethylenglycoldimethacrylat, Diethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-di-methacrylat, Trimethylolpropantrimethacrylat, aber auch Bis-GMA sowie die Reaktionsprodukte aus Isocyanaten, insbesonder Di- und/oder Triisocyanaten und OH-gruppenhaltigen Methacrylaten.

Beispiele für letztere sind die Umsetzungsprodukte von 1 Mol Hexamethylendiisocyanat mit 2 Mol 2-Hydroxyethylmethacrylat, von 1 Mol Tris-(6-isocyanatohexyl) mit 3 Mol Hydroxyethylmethacrylat, und von 1 Mol Trimethylhexamethylendiisocyanat mit 2 Mol Hydroxyethylmethacrylat. Der Anteil dieser Verbindungen in der Mischung mit dem/den Urethanacrylat(en) bewegt sich zwischen 10 und 70 Gewichtsprozent,

3

bevorzugt zwischen 20 und 50 Gewichtsprozent.

Die Verbindungen können mitan sich bekannten Katalysatoren ausgehärtet werden, die durch Strahlung aktivierbar sind. Die Verwendung eines Photosensibilisators zusammen mit einem Reduktionsmittel wird bevorzugt. Beispiele für Photosensibilisatoren sind $\alpha$-Diketone wie 9,10-Phenanthrenchinon, Diacetyl, Furil, Anisil, 4,4'-Dichlorbenzil und 4,4'-Dialkoxybenzil. Campherchinon wird bevorzugt verwendet. Beispiele für Reduktionsmittel sind Amine wie Cyanoethylmethylanilin, Dimethylaminoethylmethacrylat, n-Butylamin, Triethylamin, Triethanolamin, N,N-Dimethylanilin oder N-Methyldiphenylamin.

Sie können auch mit an sich bekannten Katalysatoren für die Heiß- oder Redoxpolymerisation ausgehärtet werden. So für die Heißpolymerisation mit Peroxiden wie Dibenzoylperoxid, Dilaurylperoxid, tert.-Butylperoktoat oder tert.-Butylperbenzoat, aber auch mit $\alpha,\alpha$-Azo-bis-(isobutyroethylester)-benzpinakol und 2,2'-Dimethylbenzpinakol.

Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind: Dibenzoylperoxid, Dilaurylperoxid und Di-4-chlorbenzoylperoxid.

Als tertiäre aromatische Amine seien beispielsweise N,N-Dimethyl-p-toluidin, Bis-(2-hydroxyethyl)-p-toluidin, Bis-(2-hydroxyethyl)-3,5-dimethylanilin und N-Methyl-N-(2-methylcarbamoyloxypropyl)-3,5-dimethylanilin genannt.

Die peroxid- bzw. aminhaltigen Monomermischungen werden bis zur Anwendung getrennt gelagert.

Die erwähnten Katalysatoren werden in Mengen von 0,01 bis 10 Gew.-%, bezogen auf das polymerisierbare Material, verwendet, insbesondere in Mengen von 0,1 bis 5 Gew.-%.

Je nach Verwendungszweck des Werkstoffes können die Materialien auch anorganische oder organische Füllstoffe enthalten. Geeignete anorganische Füllstoffe sind z. B.: Bergkristall, Kristoballit, Quarzglas, hochdisperse Kieselsäure, Aluminiumoxid und Glaskeramiken, beispielsweise Lanthan und Zirkon enthaltende Glaskeramiken (DE-A 2 347 591). Die anorganischen Füllstoffe werden zur Verbesserung des Verbundes zur Polymermatrix vorzugsweise mit einem Haftvermittler vorbehandelt. Die Haftvermittlung kann beispielsweise durch eine Behandlung mit Organosiliciumverbindungen erreicht werden (Progress in Organic Coatings 11, 297 - 308 (1983)). Bevorzugt wird 3-Methacryloyloxypropyl-trimethoxysilan eingesetzt. Es kann auch vorteilhaft sein, mehrere Füllstoffe nebeneinander einzusetzen, die einen voneinander verschiedenen Teilchendurchmesser und/oder einen unterschiedlichen Silangehalt besitzen.

Geeignete organische Füllstoffe sind z.B. Kunststoffstäube und -perlen auf Basis Polyethylen, Polypropylen, Polyvinylchlorid, Polyvinylacetat, Polystyrol, Polyamid, Polycarbonat, Polyester, Polyacrylat und Polymethacrylat.

Der Füllstoffanteil in der Mischung beträgt im allgemeinen 0 bis 80 Gew.-%, vorzugsweise 10 bis 70 Gew.-%. Dem Werkstoff können auch organische Polymere oder Copolymere zugesetzt werden. Weiterhin können auch die üblichen Hilfsstoffe wie Stabilisatoren, Lichtschutzmittel, Weichmacher, Geruchs- und Farbstoffe enthalten sein. Beispielhaft sei die folgende Zusammensetzung genannt:

100 Gewichtsteile Paste enthalten:

    a) 50 - 95 Gewichtsteile Urethanacrylat

    b) 0 - 50 Gewichtsteile Comonomer

    c) 0 - 50 Gewichtsteile Füllstoff

    d) 0 - 5 Gewichtsteile Initiator für die radikalische Polymerisation

    e) 0 - 5 Gewichtsteile Hilfsstoffe

Die erfindungsgemäßen Abformmassen werden in lichtundurchlässige Tuben oder Kartuschen abgefüllt. Bei Anwendung kann eine niedrig- oder mittel-viskose Masse mittels Spritze auf die abzuformenden Zahnreihen appliziert werden. Danach wird eine hochviskose Masse in einem Abformlöffel aus lichtdurchlässigem Material wie Polymethacrylat. Polycarbonat oder Polystyrol auf die mit der niedrig- oder mittel-viskosen Masse umspritzten Zahnreihe gedrückt. Anschließend werden die ineinandergeflossenen Massen mittels Lichtleiter durch Einwirkung von sichtbarem Licht mit einer Wellenlänge zwischen 300 und 600 Nanometer polymerisiert Man erhält ein detailgenaues Negativ der abgeformten Zahnreihe, welches anschließend mit z. B. Modellgips ausgegossen werden kann.

Neben der Verwendung als Abformmassen können die Massen sowohl in der lichthärtenden als auch in der radikalisch vernetzenden Variante als weich bleibendes Unterfütterungsmaterial für Prothesen eingesetzt werden. Da das Prothesenmaterial im allgemeinen aus Polymethacrylat besteht, ist bei Verwendung der erfindungsgemäßen Massen als Unterfütterungsmaterial ein Verbund von Prothesen- und Unterfütterungsmaterial aufgrund der gleichen chemischen Basis gegeben.

Beispiele für lichthärtende Abformmassen

Beispiel 1 (Herstellung eines Urethanmethacrylates)

4.000 g (1 Mol) eines auf Propylenglykol gestarteten Polyethers, bei dem zunächst 87 Gew.-Teile Propylenoxid, dann 13 Gew.-Teile Ethylenoxid polyaddiert wurden (OH-Zahl = 28, MG = 4.000, 25 °C = 800 mPa•s) werden im Wasserstrahlvakuum 1 Std. bei 120 °C entwässert, anschließend werden dem Ansatz 400 g (1,8 Mol) Isophorondiisocyanat und 0,1 g Zinnoctoat zugesetzt. Das Gemisch wird 1 Std. bei 120 °C unter Stickstoff gerührt, danach der NCO-Gehalt des Prepolymers (I) ermittelt (NCO gef. 1,33 %; NOC ber. 1,53 %).

Zu 600 g des auf diese Weise erhaltenen NCO-Prepolymers (I) werden 0,1 g 2,6-Di-Tert.-butyl-4-methyl-phenol, 49,8 g Glycerindimethacrylat und 0,35 Zinn(II)-octoat zugegeben. Man erwärmt die Mischung auf 60 °C und rührt das Ganze 48 Std. Danach ist kein freies NCO mehr nachweisbar. Man erhält ein tetrafunktionelles Urethanmethacrylat mit einer Viskosität von 66 Pa•s bei 20 °C.

Beispiel 2

(Herstellung eines Urethanacrylat-methacrylates)

Man verfährt wie im Beispiel 1 beschrieben, nur mit dem Unterschied, daß zu 600 g des NCO-Prepolymers (I) 0,1 g 2,6-Di-tert-butyl-4-methyl-phenol und 46,7 g Glycerinmonoacrylat-monomethylacrylat und 0,3 g Zinnoctoat zugegeben werden. Die Mischung wird 48 Std. bei 60 °C gerührt. Danach ist kein freies NCO mehr nachweisbar. Man erhält auf diese Weise ein tetrafunktionelles Urethanacrylatmethacrylat mit einer Viskosität von 237 Pa•s bei 20°C.

Beispiel 3 (Herstellung eines Urethanmethacrylates)

Man verfährt wie in Beispiel 1 beschrieben, nur mit dem Unterschied, daß zu 600 g des NCO-Prepolymers (I) 0,1 g 2,6-di-tert-butyl-4-methylphenol, 24,9 g Glycerindimethacrylat, 14,2 g Hydroxyethylmethacrylat und 0,2 g Zinn(II)-octoat gegeben werden. Die Mischung wird 48 Std. bei 60 °C gerührt. Danach ist kein freies NCO mehr nachweisbar. Man erhält auf diese Weise ein trifunktionelles Urethanmethacrylat mit einer Vikosität von 42 Pa•s bei 20°C.

Beispiel 4 (Herstellung eines Urethanmethacrylates) Vergleich

Man verfährt wie in Beispiel 1 beschrieben, nur mit dem Unterschied, daß zu 600 g des NCO-Prepolymers (I) 0,1 g 2,6-di-tert.-butyl-4-methylphenol, 28,4 g Hydroxyethylmethacrylat und 0,2 g Zinn(II)-octoat gegeben werden. Die Mischung wird 48 Std. bei 60°C gerührt. Danach ist kein freies NCO mehr nachweisbar. Man erhält auf diese Weise ein difunktionelles Urethanmethacrylat mit einer Viskosität von 58 Pa•s bei 20 °C.

Beispiel 5 (Aktivierung der Urethan(meth)acrylate)

99,45 % Urethan(meth)acrylat aus Beispiel 1, 2, 3 oder 4
0,25 % Diallylsulfonamid
0,10 % Jonol
0,20 % Campherchinon
werden 15 Stunden im Dunkeln im Rotavapor gemischt.

Beispiel 6 (Herstellung einer Abformmasse)

85,71 % Urethan(meth)acrylat aus Beispiel 1, 2, 3 oder 4, aktiviert nach Beispiel 5
14,27 % Syloid ED 5 (Fa. Grace)
0,02 % Sicomet-Farbstoff rot B 12085 (BASF)
Die Rohstoffe wurden in einem Planetenmischer im Dunkeln homogen gemischt. Die mittelviskose Abformmasse wurde in lichtundurchlässige Kartuschen abgefüllt.

Beispiel 7 (Herstellung einer Abformmasse)

60,00 % Urethan(meth)acrylat aus Beispiel 1, 2, 3 oder 4, aktiviert nach Beispiel 5
10,00 % Syloid ED 5 (Fa. Grace)
29,95 % Cristobalit-Feinstmehl
0,05 % Sicomet-Farbstoff blau P 74160 (BASF)
In einem Planetenmischer wurden die Einsatzstoffe im Dunkeln homogen gemischt. Die hochviskose Abformmasse wurde in Tuben abgefüllt.

Beispiel 8 (Prüfung der lichtgehärteten Abformmassen)

Sowohl die aktivierten Urethan(meth)acrylate aus Beispiel 5 als auch die daraus formulierten Abformmassen aus den Beispielen 6 und 7 wurden in Formen für Prüfkörper gegeben und in einem Dentacolor-Lichtofen 30 Sekunden belichtet. Danach wurde die Härte Shore A, die Dimensionsänderung nach der Spezifikation Nr. 19 der American Dental Association und die Reißfestigkeit nach DIN 53 504 gemessen. Die Ergebnisse sind in Tabelle 1 zusammengefaßt.

**Tabelle 1**

**Prüfergebnisse von lichtgehärtetem Abformmaterial**

| Masse (Beispiel) | Urethan(meth)acrylat (Beispiel) | | Härte (Shore A) [%] | Dimensionsänderung [%] | Reißfestigkeit [N/mm²] |
|---|---|---|---|---|---|
| Ungefüllt (5) | Tetrafunktionell | UMA (1) | 48 | 0,19 | 1,9 |
| | Tetrafunktionell | UAMA (2) | 47 | 0,17 | 1,7 |
| | Trifunktionell | UMA (3) | 47 | 0,22 | 1,9 |
| | Difunktionell | UMA (4) | 45 | 0,38 | 0,9 |
| Gefüllt mittelviskos (6) | Tetrafunktionell | UMA (1) | 70 | 0,11 | 6,9 |
| | Tetrafunktionell | UAMA (2) | 68 | 0,10 | 6,5 |
| | Trifunktionell | UMA (3) | 70 | 0,12 | 6,7 |
| | Difunktionell | UMA (4) | 66 | 0,31 | 4,2 |
| Gefüllt hochviskos (7) | Tetrafunktionell | UMA (1) | 82 | 0,06 | 10,9 |
| | Tetrafunktionell | UAMA (2) | 80 | 0,06 | 10,3 |
| | Trifunktionell | UMA (3) | 81 | 0,07 | 10,5 |
| | Difunktionell | UMA (4) | 78 | 0,22 | 7,2 |

**Patentansprüche**

1. Verwendung von Zubereitungen bestehend aus lichthärtenden Prepolymeren aus jeweils wenigstens einem Makrodiol, einem aliphatischen Polyisocyanat gegebenenfalls niedermolekularen Diolen bzw. Diaminkettenverlängerungen, ungesättigte Endgruppen enthaltende Monoalkohole als Kettenabbrecher und üblichen Hilfs- und Zusatzmitteln, dadurch gekennzeichnet, daß die mittlere Funktionalität der Prepolymeren bezogen auf die ungesättigen Gruppen > 2 ist, zur Herstellung von Abformmaterialien in der Dentaltechnik.

**2.** Verwendung gemäß Anspruch 1, dadurch gekennzeichnet, daß als Makrodiole Polyetherdiole des Molekulargewichtsbereichs 400 - 20.000 eingesetzt werden.

**3.** Verwendung gemäß Anspruch 1, dadurch gekennzeichnet, daß als Kettenabbrecher Hydroxyacrylate bzw. -methacrylate, Glycerindiacrylat, Glycerindimethacrylat, Pentaerythrittriacrylat, Pentaerythrittrimethacrylat bzw. gemischte Ester des Pentaerythrits mit Acrylsäure und Methacrylsäure gegebenenfalls in Abmischung mit Hydroxyethylacrylat bzw. -methacrylat eingesetzt werden mit der Maßgabe, daß die mittlere Funktionalität der Prepolymeren > 2 ist.

**4.** Verwendung gemäß Anspruch 1, dadurch gekennzeichnet, daß zum Aufbau der ungesättigten Endgruppen enthaltenden Prepolymeren aliphatische und/oder cycloaliphatische Diisocyanate, gegebenenfalls im Gemisch mit Tri- und Tetraisocyanaten eingesetzt werden mit der Maßgabe, daß die mittlere Funktionalität der Prepolymeren bezogen auf die ungesättigten Gruppen > 2 ist.

**5.** Verfahren zur Herstellung von Abformmaterialien gemäß Anspruch 1, dadurch gekennzeichnet, daß pro Mol Makrodiol des Molekulargewichtsbereichs > 400, gegebenenfalls in Abmischung mit niedermolekularen Diolen des MG Bereichs 62-400, 1,7 bis 3 Mol Diisocyanat(mischung) verwendet werden und das erhaltene Zwischenprodukt mit Hydroxyethylacrylat bzw. -methacrylat und OH-gruppenhaltiges Poly-(meth)acrylsäureestern zu Prepolymeren umgesetzt wird, deren mittlere Funktionalität bezogen auf die ungesättigten Gruppen > 2 ist.

**Claims**

**1.** Use of preparations comprising photosetting prepolymers composed of, in each case, at least one macrodiol, one aliphatic polyisocyanate, where appropriate low molecular weight diols or diamine chain extenders, unsaturated monoalcohols, containing end groups, as chain terminators and customary auxiliaries and additives, characterised in that the average functionality of the prepolymers based on the unsaturated groups is >2, for preparing impression materials in dental engineering.

**2.** Use according to Claim 1, characterised in that polyether diols of the molecular weight range 400 - 20,000 are employed as macrodiols.

**3.** Use according to Claim 1, characterised in that hydroxyacrylates or -methacrylates, glycerol diacrylate, glycerol dimethacrylate, pentaerythritol triacrylate, pentaerythritol trimethacrylate, or mixed esters of pentaerythritol with acrylic acid and methacrylic acid, where appropriate mixed with hydroxyethyl acrylate or methacrylate are employed as chain terminators, with the proviso that the average functionality of the prepolymers is >2.

**4.** Use according to Claim 1, characterised in that aliphatic and/or cycloaliphatic diisocyanates, where appropriate mixed with tri- and tetraisocyanates, are employed for building up the unsaturated prepolymers containing end groups, with the proviso that the average functionality of the prepolymers based on the unsaturated groups is >2.

**5.** Process for preparing impression materials according to Claim 1, characterised in that 1.7 to 3 mol of diisocyanate (mixture) are used per mol of macrodiol of molecular weight range >400, where appropriate mixed with low molecular weight diols of MW range 62-400, and the resulting intermediate is reacted with hydroxyethyl acrylate or methacrylate and poly(meth)acrylic esters containing OH groups to give prepolymers whose average functionality based on the unsaturated groups is >2.

**Revendications**

**1.** Utilisation de préparations constituées de prépolymères photodurcissables formés d'au moins un macrodiol, d'au moins un polyisocyanate aliphatique, le cas échéant de diols ou d'agents diaminés d'allongement de chaîne de bas poids moléculaire, des monoalcools contenant des groupes terminaux non saturés comme agents de terminaison de chaîne, et de substances auxiliaires et d'additifs classiques, caractérisée en ce que la fonctionnalité moyenne des prépolymères, rapportée aux groupes non saturés, est supérieure à 2, pour la production de matériaux pour empreintes dans l'art dentaire.

2. Utilisation suivant la revendication 1, caractérisée en ce que les macrodiols que l'on utilise sont des polyétherdiols de poids moléculaire compris dans la plage de 400 à 20 000.

3. Utilisation suivant la revendication 1, caractérisée en ce que les agents de terminaison de chaîne que l'on utilise sont des hydroxyacrylates ou -méthacrylates, le diacrylate de glycérol, le diméthacrylate de glycérol, le triacrylate de pentaérythritol, le triméthacrylate de pentaérythritol ou des esters mixtes de pentaérythritol avec l'acide acrylique et l'acide méthacrylique, le cas échéant en mélange avec l'acrylate ou le méthacrylate d'hydroxyéthyle, sous réserve que la fonctionnalité moyenne des prépolymères soit supérieure à 2.

4. Utilisation suivant la revendication 1, caractérisée en ce que des diisocyanates aliphatiques et/ou cycloaliphatiques, éventuellement en mélange avec des triisocyanates et des tétra-isocyanates, sont utilisés pour l'édification des prépolymères contenant des groupes terminaux non saturés, sous réserve que la fonctionnalité moyenne des prépolymères, rapportée aux groupes non saturés, soit supérieure à 2.

5. Procédé de production de matériaux pour empreintes suivant la revendication 1, caractérisé en ce qu'on utilise par mole de macrodiol de plage de poids moléculaire supérieure à 400, le cas échéant en mélange avec des diols de bas poids moléculaire dont la plage de poids moléculaire va de 62 à 400, 1,7 à 3 moles de diisocyanate (mélange) et le produit intermédiaire obtenu est amené à réagir avec l'acrylate ou le méthacrylate d'hydroxyéthyle et les esters d'acides poly(méth)acryliques porteurs de groupes OH pour former des prépolymères dont la fonctionnalité moyenne rapportée aux groupes non saturés est supérieure à 2.